# EUROPEAN PATENT SPECIFICATION

(11) **EP 3 427 575 B1**
(45) Date of publication and mention of the grant of the patent: **10.06.2026**
(21) Application number: 18183165.2
(22) Date of filing: 12.07.2018
(51) Int. Cl.: A01H 1/00, A01H 5/12, A01H 6/14, C12N 9/88

(54) **ENDIVE PLANT THAT SHOWS REDUCED BROWNING**
ENDIVIENPFLANZE, DIE EINE REDUZIERTE BRAUNFÄRBUNG ZEIGT
PLANTE D'ENDIVE MONTRANT UN BRUNISSEMENT REDUIT

(30) Priority: 14.07.2017 NL 2019252
(43) Date of publication of application: 16.01.2019
(73) Proprietor: Rijk Zwaan Zaadteelt en Zaadhandel B.V., 2678 KX De Lier (NL)
(72) Inventor: VAN DUN, Cornelis Maria Petrus, 2678 KX De Lier (NL)
(74) Representative: Arnold & Siedsma

(56) References cited:
- WO-A1-2008/083715
- US-A- 6 113 958
- HISAMINATO H ET AL: "Relationship between the enzymatic browning and phenylalanine ammonia-lyase activity of cut lettuce, and the prevention of browning by inhibitors of polyphenol biosynthesis", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 65, no. 5, 1 May 2001 (2001-05-01), pages 1016 - 1021, XP002428729, ISSN: 0916-8451, DOI: 10.1271/BBB.65.1016
- ABIR SALMAN ET AL: "Controlled atmosphere and heat shock affect PAL1 and HSP90 mRNA accumulation in fresh-cut endive (Cichorium intybus L.)", EUROPEAN FOOD RESEARCH AND TECHNOLOGY ; ZEITSCHRIFT F�R LEBENSMITTELUNTERSUCHUNG UND -FORSCHUNG A, SPRINGER, BERLIN, DE, vol. 227, no. 3, 26 October 2007 (2007-10-26), pages 721 - 726, XP019621733, ISSN: 1438-2385
- DATABASE WPI Week 201639, Derwent World Patents Index; AN 2016-32433C, XP002775959

## Description

The present invention relates to an endive plant *(Cichorium endivia* L.) that shows reduced browning of the leaves after the leaves having been in contact with hot water, in particular by submersion. The invention also relates to the seeds and to propagation material for obtaining such plants.

The invention further relates to a modified gene that leads to the reduced browning of the endive leaves after the leaves having been in contact with hot water, in particular by submersion, to its sequences.

The invention further relates to a molecular marker and to the use of said marker to identify a modification in the *PAL1* gene that leads to reduced browning of endive leaves having been in contact with hot water, in particular by submersion.

The invention also relates to a method for obtaining an endive plant with reduced browning of the leaves after the leaves having been in contact with hot water, in particular by submersion.

Endive *(Cichorium endivia* L.) is a flowering plant of the Asteraceae family - also known as the Compositae family - that is grown as a leafy vegetable. Endive *(Cichorium endivia* L.) belongs to the chicory genus, which also includes common chicory *(Cichorium intybus* L.) with chicory types such as radicchio, puntarelle, and Belgian endive. The edible parts of the endive plant *(Cichorium endivia* L.) are the leaves of the vegetative stage. Endive has two forms: narrow-leaved endive called curly endive or frisée, and the broad-leaved endive which is often called escarole or Scarole. The curly endive type has narrow, green, curly leaves that may be finely cut. Escarole, or broad-leaved endive, has broad green leaves. Endive is eaten like other greens, sauteed, chopped into soups and stews, or as part of a fresh salad. The outside leaves of an endive head are green and relatively bitter. The inner leaves of the endive head are light green to creamy-white and have a milder flavour. Both curly and broad-leaved endive are used in salad mixtures with lettuce that has a blander flavour to add both texture and a satisfying bitterness to the flavour of the salad.

In order to prepare the endive leaves for consumption, the leaves may also be cooked in hot water. The cooked leaves are served with cream or béchamel sauce. Consumers can cook the raw leaves, buy ready to eat meals with cooked endive leaves or buy frozen cooked endive leaves. For the consumer the green or creamy-white colour of the cooked leaves is an indicator of the quality and the freshness of the product. The most important problem occurring after the endive leaves have been in contact with hot water, is the appearance of a brown colouration on the cooked leaves. The brown colouration is unattractive to the consumer. Furthermore, the brown colouration may smear the cooking devices used to cook the endive leaves, leading to the need to clean the devices more frequently.

As a result, consumers and processing companies are looking for endive varieties comprising leaves that stay green or creamy white after contact with hot water .

Though endive *(Cichorium endivia* L.) is a popular product due to its distinctive taste, excellent shelf life and high nutritional value, at present endive leaves do not stay green or creamy white after contact with hot water, as typically endive leaves turn brown after the submerging process.

Given the need for endive plants *(Cichorium endivia* L.) with leaves that stay green or creamy-white after contact with hot water, it is the object of the present invention to provide an endive plant *(Cichorium endivia* L.) with leaves that shows reduced browning after contact with hot water.

During the research that led to the present invention a mutant endive plant (*Cichorium endivia* L.) was produced that showed reduced browning of the endive leaves after contact with hot water. The reduced browning of the leaves was found to be the result of a mutation in the *PAL1* gene.

The invention thus relates to a mutant endive plant *(Cichorium endivia* L.) comprising a modified *PAL1* gene, wherein the modification leads to reduced browning of the endive leaves after the leaves having been in contact with hot water as compared to the leaves of a plant comprising the wild type *PAL1* gene, the wild type sequence of which has a sequence similarity of 95%, 96%, 97, 98%, 99% or 100% with **SEQ ID No:1,** wherein the modification of the *PAL1* gene is a premature stop codon in the coding sequence of said gene.

The *PAL1* gene encodes the phenylalanine ammonia-lyase, herein abbreviated PAL, which is an enzyme in the phenylpropanoid pathway. Within the pathway the enzyme is responsible for the trans-cinnamate biosynthesis. It catalyzes the removal of an ammonia group from L-phenylalanine to form trans-cinnamate and ammonia. The PAL enzyme is known to be involved in browning at the wound surface of leafy vegetables. To induce this type of browning a wound surface needs to be generated by physical injuries such as cutting and punching. However, the enzyme is not known to be involved in browning of the intact endive leaves after contact with hot water.

It is surprising that the modified *PAL1* gene of the invention leads to reduced browning after contact with hot water and does not have any effect on the browning at a wound surface of an endive plant. The endive plant of the invention comprises leaves that are both attractive and tasty even after contact with hot water.

In this context endive is intended to mean *Cichorium endivia* L.

The genome of an endive plant comprises at least five *PAL* genes, that are paralogs. Paralogs or paralogous genes result from a duplication within a species and can differ in the sequences and functions. The *PAL1* gene is one of the paralogs present in the endive plant genome.

The invention further relates to a mutant endive plant *(Cichorium endivia L.)* comprising a modified *PAL1* gene, that leads to reduced browning of the endive leaves after contact with hot water, wherein the modification leads to a premature stop codon in the coding sequence of said gene and wherein the wild type sequence of the *PAL1* gene is represented by **SEQ ID No:1.**

The wild type (WT) coding sequence (CDS) of the *PAL1* gene is as identified in **SEQ ID No:1,** encoding the wild type PAL1 protein **of SEQ ID No:2.**

Disclosed herein is a modified *PAL1* gene that leads to reduced browning of the endive leaves after contact with hot water as compared to the leaves of a plant comprising the wild type *PAL1* gene, wherein the wild type *PAL1* gene has a sequence similarity of 95%, 96%, 97, 98%, 99% or 100% with the nucleotide sequence represented by **SEQ ID No:1.**

The invention further relates to a modified *PAL1* gene, the wild type sequence of which has a sequence similarity of 95%, 96%, 97, 98%, 99% or 100% with SEQ ID No:1, comprising a modification that leads to reduced browning of endive leaves after the leaves having been in contact with hot water, in particular through submersion, wherein the modification of the *PAL1* gene is a premature stop codon in the coding sequence of said gene.

The skilled person is familiar with methods for the calculation of sequence similarity. Suitably sequence similarity is calculated using EMBOSS stretcher 6.6.0 (www.ebi.ac.uk/Tools/psa/emboss stretcher), using the resulting "similarity score".

Disclosed herein is a mutant endive plant *(Cichorium endivia* L.) comprising a modified *PAL1* gene, wherein the modification leads to reduced browning of the endive leaves after contact with hot water and wherein the modification is the introduction of a premature stop codon. The invention relates to an endive plant of the invention, wherein the premature stop codon is caused by the presence of a SNP form G to A at position 66 of **SEQ ID No:1.**

Also, disclosed herein is a mutant endive plant *(Cichorium endivia* L.) comprising a modified *PAL1* gene, wherein the genetic modification leads to reduced browning of the endive leaves after contact with hot water and wherein the genetic modification is the introduction of a premature stop codon.

Further disclosed herein is a modified *PAL1* gene, the wild type sequence of which has a sequence similarity of 95%, 96%, 97, 98%, 99% or 100% with SEQ ID No:1, comprising a genetic modification that leads to reduced browning of endive leaves after the leaves having been in contact with hot water, in particular through submersion.

Premature stop codons typically lead to the expression of a truncated version of the encoded protein. Depending on the position of the mutation in the coding sequence, a truncated version of a protein may lack one or more domains that are essential to perform its function and/or to interact with substrates or with other proteins, and/or it may lack the ability to fold properly into a functional protein. Substitutions, deletion or addition of nucleotides within a gene sequence may lead directly to the formation of a premature stop codon or to a frame shift mutations. Frame shift mutations are caused by the introduction or deletion of one or more base pairs in a DNA sequence encoding a protein. When the number of inserted or deleted base pairs at a certain position is not a multiple of 3, the triplet codon encoding the individual amino acids of the protein sequence become shifted relative to the original open-reading frame, and the encoded protein sequence changes dramatically. Protein translation will result in an entirely different amino acid sequence than that of the originally encoded protein, and often a frame shift also leads to a premature stop codon in the open reading frame. The overall result is that the encoded protein no longer has the same biological function as the originally encoded protein.

The modification of the *PAL1* gene according to the invention that is responsible for the reduced browning of the endive leaves after contact with hot water, leads at the protein level at position 22 of **SEQ ID No:2** to the conversion of a Tryptophan to a premature stop codon responsible for the truncated version of the PAL1 protein.

The CDS sequence of the modified *PAL1* gene of the invention comprising the SNP form G (guanine) to A (adenine) at position 66 of **SEQ ID No:1** is as identified in **SEQ ID No:3.**

Disclosed herein is a plant comprising a modified *PAL1* gene represented by **SEQ ID No:3** and leading to reduced browning of the endive leaves after contact with hot water as compared to the leaves of a plant comprising the wild type *PAL1* gene, wherein the wild type *PAL1* gene is represented by **SEQ ID No:1.**

Additionally, disclosed herein is a modified *PAL1* gene represented by **SEQ ID No:3** and leading to reduced browning of the endive leaves after contact with hot water as compared to the leaves of a plant comprising the wild type *PAL1* gene, wherein the wild type *PAL1* gene is represented by **SEQ ID No:1.**

The invention further relates to an endive plant comprising a modified *PAL1* gene of the invention that leads to reduced browning of the endive leaves after contact with hot water as compared to the leaves of a plant comprising the wild type *PAL1* gene, wherein hot water is a water with a temperature of 85°C to 95°C, preferably about 90°C.

Furthermore, disclosed herein is an endive plant comprising a modified *PAL1* gene of the invention that leads to reduced browning of the endive leaves after contact with hot water for 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89 or 90 seconds, as compared to the leaves of a plant comprising the wild type *PAL1* gene, wherein hot water is a water with a temperature of 85°C, 86°C, 87°C, 88°C, 89°C, 90°C, 91°C, 92°C, 93°C, 94°C or 95°C.

Also, disclosed herein is an endive plant comprising a modified *PAL1* gene that leads to reduced browning of the endive leaves after contact with hot water for 60 seconds, as compared to the leaves of a plant comprising the wild type *PAL1* gene, wherein hot water is a water with a temperature of 90°C.

The trait of the present invention is displayed in any endive plant of the invention comprising the modified *PAL1* gene. The said endive plant of the invention may be identified phenotypically, e.g. when grown under normal growing conditions in the greenhouse or on the field, after contact with hot water and comparing the colour of the leaves to the wild type endive leaves. No endive plants existed in the prior art which show reduced browning of the leaves after contact with hot water. Endive plants of the invention include endive plants carrying the modified *PAL1* gene in a heterozygous state and endive plants carrying the modified *PAL1* gene in a homozygous state.

Disclosed herein is a modified *PAL1* gene that leads to reduced browning of endive plant leaves after contact with hot water, wherein the wild type sequence of said gene is represented by **SEQ ID No:1.**

Additionally, disclosed herein is a modified *PAL1* gene that leads to reduced browning of endive plant leaves after contact with hot water, wherein the wild type sequence of said gene is represented by **SEQ ID No:1** and wherein the modification results in a premature stop codon.

Further disclosed herein is a modified *PAL1* gene that leads to reduced browning of endive plant leaves after contact with hot water, wherein the wild type sequence of said gene is represented by **SEQ ID No:1** and wherein the modification results in a premature stop codon caused by the presence of a SNP form G to A at position 66 of **SEQ ID No:1.**

Disclosed herein is a modified *PAL1* gene that can be introgressed from an endive plant comprising the modified *PAL1* gene into a plant lacking the modified *PAL1* gene but having other desired traits, using crossing when the plants are sexually compatible, optionally combined with techniques that aid the development of viable seeds or facilitate development into a plant. Use of the modified *PAL1* gene of the invention comprises use by introgressing the gene from a donor plant comprising the modified *PAL1* gene and showing reduced browning of the endive leaves after contact with hot water, into a recipient plant that lacks the modified *PAL1* gene. The introgression of the modified *PAL1* gene leads to the phenotype of the invention.

The wild type endive plant into which the trait of the invention may be introduced is an endive plant of any type.

The phenotype of the invention can also be introduced into other plants belonging to the Cichorium family such as chicory and radicchio plants (*Cichorium intybus* L.).

The modified *PAL1* gene of the invention can be introgressed from an endive plant *(Cichorium endivia* L.) comprising the modified *PAL1* gene of the invention into an endive plant *(Cichorium endivia* L.) lacking the modified *PAL1* gene using standard breeding techniques.

The modified *PAL1* gene of the invention can also be introgressed from an endive plant *(Cichorium endivia* L.) comprising the modified *PAL1* gene of the invention into a chicory or raddichio plant *(Cichorium intybus* L.) lacking the modified *PAL1* gene using standard breeding techniques.

The modified *PAL1* gene of the invention can be introgressed from a chicory or raddichio plant *(Cichorium intybus* L.) comprising the modified *PAL1* gene of the invention into a chicory or raddichio plant *(Cichorium intybus* L.) lacking the modified *PAL1* gene using standard breeding techniques.

Also, the modified *PAL1* gene of the invention can be introgressed from a chicory or radicchio plant *(Cichorium intybus* L.) comprising the modified *PAL1* gene of the invention into an endive plant *(Cichorium endivia* L.) lacking the modified *PAL1* gene using standard breeding techniques.

Selection for endive plants that have obtained the trait of the invention from an endive plant carrying the modified *PAL1* gene is started in the F1 or any further generation from a cross between the recipient plant and a donor plant, by comparing the presence and the intensity of the brown colouration of the plant assumed to carry the modified *PAL1* gene to the brown colouration of the recipient plant before receiving the modified *PAL1* gene after contact with hot water. Suitably the endive plants that have obtained the trait can also be selected by using a molecular marker that is based upon the modification to the *PAL1* gene that underlies the trait of the invention.

Alternatively, selection for the modified *PAL1* gene is started in the F2 or any further generation of a cross or alternatively of a backcross. Selection of plants in the F2 can optionally be done phenotypically based on the observation of the brown colouration after contact with hot water, as well as by using a molecular marker(s) which directly or indirectly detect(s) the modification of the *PAL1* gene that underlies the trait.

Selection for plants having the modified *PAL1* gene, which when heterozygously or homozygously present leads to the reduced browning, can also be started in the F3 or a later generation.

Crossing can optionally be followed by embryo rescue techniques or other techniques that result in a successful combination and introgression, which techniques are known to the person skilled in the art.

The endive plants comprising the modified *PAL1* gene of the invention can be phenotypically selected after submerging one or more leaves of this plant or bringing one or more leaves of this plant into contact with in hot water and comparing the presence and intensity of the brown colouration of the leaves with the brown colouration of one or more leaves taken from a wild type endive plant.

In order to perform the comparison, leaves of the plant of the invention and leaves of the wild type plant need to be submerged in or brought into contact with hot water. For this process, the leaf samples are preferably two or three young leaves collected from the center of the endive plants. The leaves in the heart of an endive plant are usually lighter green or even yellow. Even more preferably the phenotypical analysis is performed with two leaves that are the last green leaves of the heart of the endive plant.

The leaves can be put into a closed plastic bag such as a plastic zipper storage bag and as much air as possible is removed by hand from the bag. Two or three bags containing leaves can be simultaneously submerged in the hot water. The water temperature of the water bath is 85°C, 90°C or 95°C and the bags are preferably completely submerged in the water bath for about 30 seconds, 60 seconds or 90 seconds.

After the this process the leaves are taken out of the plastic bags and laid on a surface such as filter paper in order to compare the presence and the intensity of the brown colouration of the leaves.

The endive leaves of the plant of the invention that comprises the *PAL1* gene of the invention, show reduced browning as compared to the leaves from the wild type plant. The difference of the brown colouration of the leaves is represented in **Figures 3** and **4.**

The invention further relates to seed comprising the modified *PAL1* gene of the invention, wherein the plant grown from the seed shows reduced browning of the endive leaves after contact with hot water, in particular through submersion.

A seed of the invention may comprise the modified *PAL1* gene heterozygously or homozygously. Preferably, a seed of the invention comprises a modified *PAL1* gene homozygously.

In endive plants, the modified *PAL1* gene of the invention is inherited in a semi-dominant or incomplete dominant way. When the modified *PAL1* gene is homozygously present, the progeny plant displays the trait of the invention in the same or in a similar way as the plant grown from seed of which a representative sample was deposited under accession number NCIMB 42378. This means that such progeny shows a strongly reduced browning or do not show any browning of the leaves after contact with hot water as compared to a wild type plant. When the modified *PAL1* gene of the invention is heterozygously present, the progeny plant displays the trait of the invention in a way that is between the wild type endive plant and an endive plant comprising the modified *PAL1* gene of the invention in a homozygous state. The progeny plant that comprises the modified *PAL1* gene of the invention in a heterozygous state shows a reduced browning of the endive leaves after contact with hot water as compared to the wild type endive plant, but a light brown colouration is still present on the leaves.

Disclosed herein is a progeny of an endive plant of the invention, wherein the progeny plant comprises the modified *PAL1* gene, and which progeny plant shows reduced browning of the endive leaves after contact with hot water.

In addition to this, the plant may be modified in one or more other characteristics. Such additional modifications are for example effected by mutagenesis or by transformation with a transgene.

A modified *PAL1* gene of the invention can alternatively be introduced into a plant by a transgenic method or a cisgenic method. Use of a modified *PAL1* gene of the invention for producing a plant that shows reduced browning of the endive leaves after contact with hot water comprises the introduction of a modified exogenous *PAL1* gene by a transgenic or a cisgenic method.

The invention relates to a molecular marker comprising a SNP at position 66 of **SEQ ID No:1,** in particular a SNP from G to A.

The skilled person is familiar with creating and using molecular markers to identify such modifications. Examples of suitable molecular markers are markers to identify the single-nucleotide polymorphism (SNP) from G to A in the *PAL1* gene of the endive plant according to **SEQ ID No. 1.** Such a marker can be a SNP marker comprising the SNP that is to be identified, or any other molecular marker that the skilled person can design based on a modification of the genome that underlies the trait. Other routinely used molecular markers for the identification of variation or modifications of the genome are for example, but not limited to, RFLPs, SSLPs, AFLPs, RAPDs, VNTRs, SSRs, STRs, DArTs, and RAD markers.

The invention further relates to the use of the molecular marker comprising a SNP at position 66 of **SEQ ID No:1,** in particular a SNP from G to A, to identify a modification in the *PAL1* gene which leads to reduced browning of the endive leaves after contact with hot water, in particular through submersion. Such a marker can be a molecular marker comprising the SNP that is to be identified, or another marker that the skilled person can easily design when the position of the modification to be identified is known.

The invention further relates to parts of an endive plant of the invention that are suitable for sexual and/or vegetative reproduction, i.e. propagation material, capable of developing into and/or being derived from a plant of the invention, wherein the propagation material comprises the modified *PAL1* gene of the invention and wherein the propagation material capable of growing into and/or being derived from a plant of the invention is selected from the group consisting of microspores, pollen, ovaries, ovules, embryos, embryo sacs, egg cells, cuttings, roots, root tips, hypocotyls, cotyledons, stems, leaves, flowers, anthers, seeds, meristematic cells, protoplasts, callus, and cells, or a tissue culture thereof.

Disclosed herein is a propagation material that is formed by a seed of an endive plant of the invention, wherein the seed is capable of developing into a plant that comprises the modified *PAL1* gene in a heterozygous or homozygous state.

The plant developed out of the propagation material comprises the modified *PAL1* gene of the invention.

The invention also relates to a leaf of an endive plant comprising the modified *PAL1* gene of the invention.

Disclosed herein is a food product comprising one or more harvested parts of an endive plant comprising the modified *PAL1* gene of the invention. The harvested part or food product can be or comprises a stem, a leaf, a petiole, or any other part of an endive plant, and is or comprises preferably a leaf or part thereof. A food product comprising parts of the endive plant of the invention is for example a salad, wherein the endive leaves may optionally be mixed with other leaves of for example lettuce, spinach, beet, Swiss chard, etc. The food product or harvested part, may have undergone one or more processing steps. Such a processing step might comprise but is not limited to any one of the following treatments or combinations thereof: cutting, washing, cooking, steaming, baking, frying, pasteurizing, freezing, grinding, extracting oil, pickling, or fermenting. The processed form that is obtained is also part of this invention. The processed endive may also be included in another food product, such as pie, stew, soup or a pasta product. Such food product will usually be pre-packed and is intended for sale in a supermarket. Also disclosed herein is the use of endive plants of the invention or parts thereof in the preparation of food products, in particular salads, pies, stews, soups and pastas.

Further, disclosed herein is aa container comprising one or more harvested parts of an endive plant comprising the modified *PAL1* gene of the invention, in particular intended for being brought in contact with hot water. The harvested part or food product can be or comprises a stem, a leaf, a petiole, or any other part of an endive plant, and is or comprises preferably a leaf or part thereof.

In addition, disclosed herein is a container comprising one or more endive plants of the invention for harvest of leaves from the endive plant of the invention in a domestic environment. Optionally, said container further comprises a growth substrate.

The invention also relates to a method for obtaining an endive plant with reduced browning of the leaves after contact with hot water, comprising reducing the endogenous level of the PAL1 protein in the plant, wherein the wild type sequence of the PAL1 protein has a sequence similarity of 95%, 96%, 97, 98%, 99% or 100% with the protein sequence represented by SEQ **ID** No: 2

The level of expression of a PAL1 protein can be measured by analysis of the *PAL1* gene expression for instance by RT-PCR or by quantification of the PAL1 protein level for instance with antibodies.

The invention further relates to a method for obtaining an endive plant with reduced browning of the leaves after contact with hot water, comprising reducing the endogenous level of the PAL1 protein in the plant by mutation of the *PAL1* gene.

Mutagenic treatments are for example chemicals and/or irradiation treatments. Some mutagenic treatment are used to perform a random introduction of at least one modification by means of one or more chemical compounds, such as ethyl methanesulphonate (EMS), nitrosomethylurea, hydroxylamine, proflavine, N-methyl-N-nitrosoguanidine, N-ethyl-N-nitrosourea, N-methyl-N-35 nitro-nitrosoguanidine, diethyl sulphate, ethylene imine, sodium azide, formaline, urethane, phenol and ethylene oxide, and/or by physical means, such as UV-irradiation, fast-neutron exposure, X-rays, gamma irradiation, and/or by insertion of genetic elements, such as transposons, T-DNA, retroviral elements. Mutagenic treatment also comprises the more specific, targeted introduction of at least one modification by means of homologous recombination, oligonucleotide-based mutation induction, zinc-finger nucleases (ZFNs), transcription activator-like effector nucleases (TALENs) or Clustered Regularly Interspaced Short Palindromic Repeat (CRISPR) systems.

Modified PAL1 genes can be obtained and/or identified by use of TILLING.

Disclosed herein is a mutant endive plant *(Cichorium endivia* L.) comprising a modified *PAL1* gene, wherein the induced modification leads to reduced browning of the endive leaves after the leaves having been in contact with hot water, in particular by submersion, as compared to the leaves of a plant comprising the wild type *PAL1* gene.

When the expression of the *PAL1* gene is reduced, the expression of the modified *PAL1* gene is less than the expression of the wild type *PAL1* gene or is completely prevented and thus absent.

In general, gene expression may be reduced or even prevented by means of preventing the transcription of the gene. Preventing transcription may for example be achieved by means of RNA oligonucleotides, DNA oligonucleotides or RNAi molecules directed against the *PAL1* gene promoter, or preferably by means of the expression of a negatively acting transcription factor acting on the *PAL1* gene promoter. Gene expression may also be reduced or prevented by destabilizing *PAL1* mRNA or transcript, preferably by means of nucleic acid molecules that are complementary to the *PAL1* mRNA or transcript, selected from the group consisting of antisense RNA, RNAi molecules, Virus-Induced Gene Silencing (VIGS) molecules, co-suppressor molecules, RNA oligonucleotides or DNA oligonucleotides. Such methods for destabilizing mRNA or transcripts are well known to the person skilled in the art.

Disclosed herein is the use of the endive plant comprising a modified *PAL1* gene of the invention, as a donor plant of the modified *PAL1* gene in a breeding program for the development of endive plants showing reduced browning of the endive leaves after contact with hot water.

Also disclosed herein is the use of the plant of the invention, a plant produced from the seed of the invention or from the propagation materials of the invention, as germplasm in a breeding program for the development of endive plants showing reduced browning of the endive leaves after the leaves having been in contact with hot water, in particular through submersion.

As used herein a "mutant endive plant", is an endive plant that comprises a modified *PAL1* gene, wherein the modification is effected by mutagenic treatment such as CRISPR, by a chemical agent, by radiation, or a combination thereof. The term "mutant endive plant" encompasses herein also endive plants that have a reduced endogenous level of the PAL1 protein accomplished by methods such as gene silencing or RNAi.

As used herein the phrase "after contact with hot water" or "after having been in contact with hot water" is intended to encompass all forms of contact of the leaves with hot water, i.e. physically touching the leaf with hot water, such as dipping, spraying etc. In particular, the phrase encompasses submersion in hot water.

In this application, the term "modification" refers to changes to the wild type *PAL1* gene that leads to a "modified" version of the wild type gene called herein "modified *PAL1* gene". The terms "modification" and "mutation" may be used interchangeably. A "gene" in the context of this application comprises exonic sequences and regulatory sequences such as a promoter sequence and if present also comprises intronic sequences. Modifications to the *PAL1* gene and/or regulatory sequences thereof, may inhibit gene transcription such that the expression of the modified gene is reduced or prevented. Modifications may also be changes to the sequence of the *PAL1* gene and/or regulatory sequences thereof that lead to a reduced level, reduced activity or complete absence of the encoded PAL1 protein.

As used herein "the modified *PAL1* gene of the invention" or "the *PAL1* gene of the invention" is the endive *PAL1* gene that comprises any modification that leads to reduced browning of the leaves after contact with hot water, in particular the substitution of a G to an A in **SEQ ID No:1** at position 66, leading to a premature stop codon in the coding sequence of said gene, wherein the wild type sequence of the *PAL1* gene is represented by **SEQ ID No:1.** Alternative mutations introducing a premature stop codon and leading to the phenotype of reduced browning of the leaves after contact with hot water are also part of this invention.

In the context of this application, the "trait of the invention" refers to the reduced browning phenotype of the endive leaves after contact with hot water. "Trait of the invention", "trait" or "phenotype of the invention" may be used interchangeably.

A plant that comprises the modified *PAL1* gene of the invention that leads to reduced browning of the leaves after contact with hot water is referred to herein as "plant of the invention" or "endive plant of the invention". After contact with hot water the leaves of the plant of the invention show a reduced browning as compared to the leaves of the wild type plant.

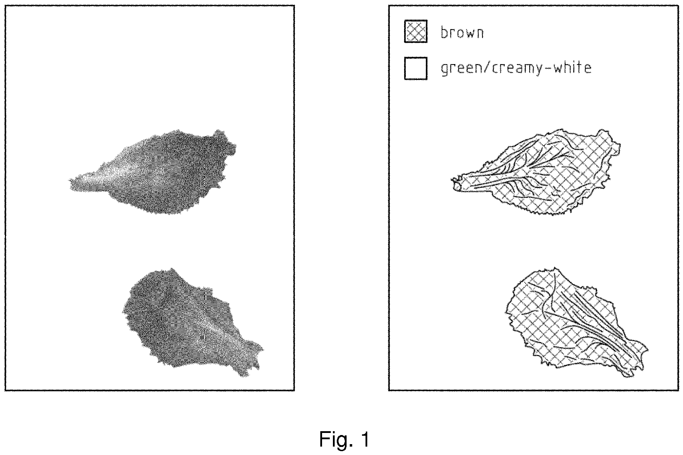

As used herein "browning" is the appearance of a brown colouration of the endive leaves of an endive plant after the leaves having been in contact with hot water, in particular by submersion i.e. the colour of the leaves or parts thereof turns from green or creamy-white to brown. The brown pigments appear on parts of the endive leaves or the endive leaf as a whole, after having been in contact with, hot water for a certain time. Examples of endive leaves that show browning are represented by the wild type in **Figure 1****.** The brown colouration appears when the endive leaves are exposed to the air and is therefore probably related to an oxidation process.

The term "reduced browning" of the leaves is always measured in relation to the browning of a leaf of a wild type plant that carries a wild type *PAL1* gene. A wild type *PAL1* gene does not comprises the modification that consist in the substitution of G with A and is represented by **SEQ ID No:1.** The wild type gene encodes the wild type protein that is represented by **SEQ ID No:2.** An endive plant comprising leaves that show reduced browning, comprises leaves that have a less intense brown colour and stay greener or more creamy-white after contact with hot water. Preferably the leaves do not show any browning and stay green or creamy white. To be comparable the leaves of the wild type plant serving as a control plant that carries a non-modified *PAL1* gene and leaves of the plant of the invention carrying a modified *PAL1* gene should be the same type of leaves (inner or outer leaves), similarly sized, of a similar developmental stage, originating from plants of the same type of endive that have grown up under comparable conditions (light, water, soil, temperature) and should have a comparable intensity of green color. Preferably, the comparison is performed with young leaves. More preferably, the comparison is performed with the last green leaves from the center (heart) of the endive head. To compare the browning of the leaves, the leaves of the endive plant of the invention and of a wild type endive plant should be stored under the same conditions before physically touching the hot water and be brought into contact with the water under the same conditions, in particular in hot water having the same temperature and during the same amount of time.

As used herein the word "progeny" is intended to mean the offspring or the first and all further descendants from a cross with a plant of the invention that shows the trait of the invention and carries the modified *PAL1* gene underlying the trait. Such progeny is for example obtainable by crossing a first endive plant with a second endive plant, wherein one of the plants was grown from seeds of which a representative sample was deposited under accession number NCIMB 42378, but may also be the progeny of any other endive plant carrying the modified *PAL1* gene of the invention.

### DEPOSIT INFORMATION

Seeds of an endive plant *(Cichorium endivia L.)* 11R.30758 that comprises the modified *PAL1* gene of the invention, were deposited with NCIMB Ltd, Ferguson Building, Craibstone Estate, Bucksburn, Aberdeen AB21 9YA, UK, on 26 February 2015 under deposit accession number NCIMB 42378. Seeds of these deposits comprise the modified *PAL1* gene of the invention in a homozygous state. Plants grown from these seeds thus show reduced browning of the endive leaves after the leaves having been in contact with hot water, in particular by submersion.

The deposited seeds do not meet the DUS criteria which are required for obtaining plant variety protection, and can therefore not be considered to be plant varieties.

### FIGURES

The invention will be further illustrated in the examples that follow and that are only given for illustrative purposes and in no way intended to be limiting on the scope of the invention. In the examples reference is made to the following figures:
**Figure 1****:** shows a picture and a schematic representation of two leaves of a wild type endive plant. The leaves were submerged for 60 seconds in water having a temperature of 90°C. The shading in the schematic representation indicates the brown colouration.
**Figure 2****:** shows a picture and a schematic representation of two leaves of an endive comprising the modified *PAL1* gene of the invention in a homozygous state. The leaves were submerged for 60 seconds in water having a temperature of 90°C. The shading in the schematic representation indicates the brown colouration.

### EXAMPLES

### EXAMPLE 1

### Creation of the plants of the invention

A mutant endive plant *(Cichorium endivia* L.), having a modified version of the *PAL1* gene, was obtained by means of chemical mutagenesis and subsequent TILLING screening of about 1550 M2 plants. DNA sequencing revealed the presence of a G to A mutation (TGG to TGA) in one M2 plant, leading at the protein level to the conversion of a Tryptophan to a premature stop codon at position 22 of **SEQ ID No:2,** and hence to the presumed expression of a truncated version of the PAL1 protein **(SEQ ID No:4)** in this mutant plant.

Phenotypically this mutant lettuce plant showed a reduced browning of the leaves as compared to the wild type endive leaves, however a light brown colouration was still present on the leaves. When the plant was self-pollinated and allowed to produce seeds, the homozygous mutant plants of the next generation showed a strong reduction or even absence of the browning after contact with hot water.

### EXAMPLE 2

### Phenotypical analysis of the plants of the invention

Endive plants of the invention comprising the modified *PAL1* gene that leads to reduced browning of the endive leaves after contact with hot water, were identified by comparing the presence and the intensity of the brown colouration to the colouration of the wild type plant leaves.

In order to compare the browning of leaves from an endive wild type plant and the plant of the invention, leaf samples were taken from both type of plants and submerged in hot water. The wild type plant does not comprise the modified *PAL1* gene that leads to reduced browning of the leaves after submerging the leaves in hot water and the plant of the invention comprises a modified *PAL1* gene, wherein the modification consists in the substitution of G with A on position 66 of **SEQ ID No:1.**

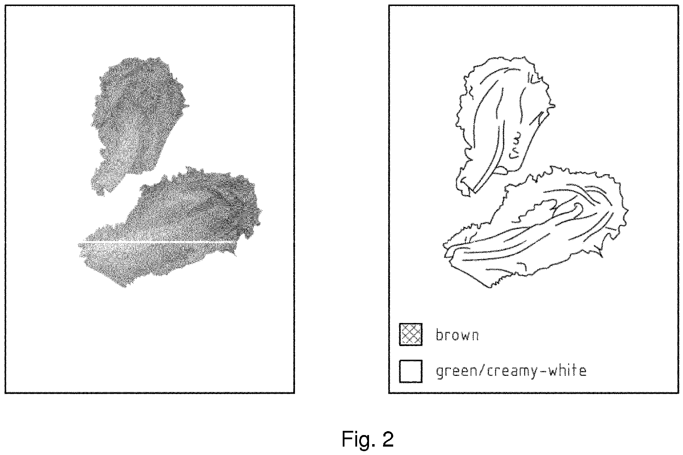

Two young green leaves were taken from the endive plant of the invention and a wild type endive plant. For the phenotypical analysis the leaves to test were taken from the last green leaves of the heart of the endive plant heads. The leaves were put into plastic zipper storage bags and as much air as possible was removed by hand from the bags. The plastic bags comprising the endive leaves were submerged for 60 seconds in a hot water bath having the temperature of 85°C, 90°C and 95°C. Two or three bags were simultaneously submerged in the water bath. After 30 seconds, 60 seconds or 90 seconds the plastic bags were taken out of the water bath and opened. The leaves were taken out of the plastic bags and laid on filter paper in order to compare their colours. For all the herein tested combinations of temperature and time, the leaves of the wild type endive plant had an intense brown colouration and the leaves of the mutant plant comprising the mutation of the invention homozygously showed a reduced brown colouration as compared to the wild type leaves. An example of the difference of the colouration of endive leaves incubated for 60 seconds in hot water with a temperature of 90°C is represented in **Figures 1** and **2****.** The leaves of the wild type endive plant have an intense brown colouration **(****Figure 1****)** and the leaves of the endive plant comprising the *PAL1* in a homozygous state do not show any browning **(****Figure 2****).**

### EXAMPLE 3

### Transfer of the trait of the invention to other endive plants

Endive plants of the deposit NCIMB 42378, comprising the modified *PAL1* gene of the invention in a homozygous state, called donor plants, were crossed with wild type endive plants, called the recipient plants and which do not carry the trait of the invention and therefore do not show any reduced browning of the endive leaves after submerging the leaves in hot water. The F1 plants resulting from this cross had a phenotype that was intermediate between the wild type parent and the endive plant comprising the modified *PAL1* gene in a homozygous state. This suggests a semi-dominant inheritance of the trait of the invention. These F1 plants had leaves that showed a reduced browning as compared to the wild type endive plant but the brown colouration is less reduced as compared to the endive plant comprising the modified *PAL1* gene in a homozygous state. The presence of the trait of the invention in a heterozygous state could also be detected by means of a molecular marker. The SNP used for this purpose is the SNP at position 66, wherein the SNP is G to A.

Subsequently, these F1 plant were self pollinated and F2 plants were obtained. The different F2's segregated in a manner that corresponds with a monogenic semi-dominant, also called incomplete dominant, inheritance of the trait of the invention. The result in the F2 was approximately one plant with the wild type parent phenotype to one plant with reduced browning phenotype of the donor parent to two plants with an intermediate phenotype between the wild type parent and the donor parent.

### SEQUENCES

**SEQ ID No: 1** is the wild type CDS sequence of the *PAL1* gene of an endive plant *(Cichorium endivia L.).*
**SEQ ID No: 2** is the encoded wild type PAL1 protein of an endive plant (*Cichorium endivia L.).*
**SEQ ID No: 3** is the CDS sequence of the modified *PAL1* gene of the invention, wherein the modification located at position 66 and consists in a substitution of a G by a A.
**SEQ ID No:4** is the modified, i.e. truncated, version of the PAL1 protein of the invention.

**SEQ ID No:1**
   *> Cichorium endivia L. PAL1* WT gene 
ATGGAGAACGGTAATCATGTTAATGGAGTCGTTAAGGAGTTTTGCATCAAGGATCCGT
TGAACTGGGGGGTTGCGGCGGAGGCGTTGACCGGGAGTCACCTTGATGAGGTGAAGA
AGATGGTGAAGGAGTTCAGGAAGCCGGTGGTTAAGCTTGGAGGAGAGACACTGACGG
 
TGTCTCAAGTGGCGGGGATCGCAGCTGCTAATGACAGCACCACCGTGACGGTGGAGCT
GTCGGAGGCGGCGAGGGCTGGAGTGAAGGCGAGCAGTGATTGGGTTATGGAGAGCAT
GAACAAAGGGACTGATAGTTATGGTGTTACCACCGGCTTCGGCGCCACCTCTCACCGG
AGAACCAAGCAAGGCGGTGCTTTACAGAAGGAGCTCATTAGATTTTTGAACGCCGGA
ATATTCGGTAATGGAACGGAATCTAGCCACACCCTACCACACTCAGCCACAAGAGCCG
CCATGCTTGTCAGAATCAACACCCTTCTCCAGGGATACTCCGGCATTCGATTCGAGAT
CTTGGAAGCTATCACCAAGTTCCTCAATAACAACATCACTCCTTGTTTACCCCTCCGTG
GGACCATCACCGCCTCCGGTGACCTTGTTCCATTATCTTACATCGCTGGTCTCCTCACC
GGCCGCCCAAACTCAAAGGCCGTCGGTCCAACCGGAGAAATCCTCAATGCCGAAAAG
GCATTCGCTGCTGCCGGTGTCGAAGGTGGTTTCTTCGAGTTACAGCCTAAAGAAGGGC
TAGCACTTGTTAACGGCACCGCCGTGGGATCCGGGATGGCGTCGATGGTTCTCTTCGA
AGCTAATGTACTTGCGTTGTTGTCGGAAGTGTTATCGGCGATCTTCGCTGAAGTTATGC
AAGGGAAGCCGGAGTTCACCGACCACTTAACACACAAATTGAAGCACCACCCCGGCC
AAATCGAAGCCGCCGCCATCATGGAATACATTTTGGACGGAAGCGATTACGTCAAGG
CGGCGCAAAAGGTACACGAAATGGACCCCTTACAGAAACCAAAACAAGACCGTTACG
CTCTCCGGACATCGCCCCAATGGCTCGGACCACACATTGAAGTAATCCGATCATCAAC
CAAAATGATCGAGAGGGAAATCAACTCCGTCAACGACAATCCACTCATCGACGTTTCC
AGAAACAAAGCCTTACACGGTGGCAACTTCCAAGGAACTCCAATCGGTGTTTCCATGG
ACAACACCCGTCTAGCCATCGCTTCCATCGGAAAACTCATGTTCGCCCAATTTTCTGAA
CTCGTTAACGATTTCTACAACAATGGATTACCGTCAAATCTCTCCGGCGGTCGTAACCC
TAGCTTGGATTACGGGTTCAAAGGTGGAGAAATCGCCATGGCTTCTTATTGCTCGGAG
CTCCAATTTCTCGCGAACCCAGTCACCAACCACGTTCAAAGCGCCGAACAACACAATC
AAGACGTTAACTCTTTGGGCTTAATTTCAGCGAGAAAAACTCAAGAAGCTGTCGAGAT
CTTAAAACTCATGTCCTCCACATATTTGGTCGCTCTATGCCAATCCATCGATTTACGCC
ATTTGGAAGAAAACATGAAATCGACAGTGAAGAACACCGTCAGCCAAGTCGCGAAAA
AGGTCCTAACAATGGGCGTCAACGGCGAGCTCCACCCTTCCAGATTCTGCGAAAAGGA
TTTACTCCGTGTTGTTGATCGCGAGTACGTCTTCGCCTACATCGACGACGCTTGCAGCG
CCACCTACCCGTTAATGCAAAAGCTCCGTCAAGTTCTCGTCGACCACGCCCTAAACAA
CGGCGAAACAGAGAAGAACACCAGCACCTCCATTTTCCAAAAGATCGCCACCTTCGA
AGACGAATTAAAAGCACTTTTACCGAAAGAAGTAGAAGCTGTCAGAATCGCGTACGA
AAACGACACATTACCGATCGCGAACAGAATCAAAGCCTGCAGATCGTACCCGTTGTAC
AGGTTTGTCAGGGAGGAGCTCGGCGGAGTGTTTTTGACCGGCGAGAAGGTGACGTCG
CCAGGGGAGGAGTTCGACAAGGTGTTTACCGCCATGTGCAAGGGTCAAATCATCGATC
CGTTGTTGGAATGTGTTGAAGGCTGGAATGGTGAACCTCTTCCGATTTGTTAG
**SEQ ID No:2**
   *> Cichorium endivia* L. PAL1 protein 
MENGNHVNGVVKEFCIKDPLNWGVAAEALTGSHLDEVKKMVKEFRKPVVKLGGETLTV
SQVAGIAAANDSTTVTVELSEAARAGVKASSDWVMESMNKGTDSYGVTTGFGATSHRRT
KQGGALQKELIRFLNAGIFGNGTESSHTLPHSATRAAMLVRINTLLQGYSGIRFEILEAITKF
LNNNITPCLPLRGTITASGDLVPLSYIAGLLTGRPNSKAVGPTGEILNAEKAFAAAGVEGGF
FELQPKEGLALVNGTAVGSGMASMVLFEANVLALLSEVLSAIFAEVMQGKPEFTDHLTHK
LKHHPGQIEAAAIMEYILDGSDYVKAAQKVHEMDPLQKPKQDRYALRTSPQWLGPHIEVI
RSSTKMIEREINSVNDNPLIDVSRNKALHGGNFQGTPIGVSMDNTRLAIASIGKLMFAQFSE
LVNDFYNNGLPSNLSGGRNPSLDYGFKGGEIAMASYCSELQFLANPVTNHVQSAEQHNQ
DVNSLGLISARKTQEAVEILKLMSSTYLVALCQSIDLRHLEENMKSTVKNTVSQVAKKVL
TMGVNGELHPSRFCEKDLLRVVDREYVFAYIDDACSATYPLMQKLRQVLVDHALNNGET
EKNTSTSIFQKIATFEDELKALLPKEVEAVRIAYENDTLPIANRIKACRSYPLYRFVREELGG
VFLTGEKVTSPGEEFDKVFTAMCKGQIIDPLLECVEGWNGEPLPIC
**SEQ ID No:3**
   *> Cichorium endivia* L. modified *PAL1* gene 
ATGGAGAACGGTAATCATGTTAATGGAGTCGTTAAGGAGTTTTGCATCAAGGATCCGT
TGAACTGAGGGGTTGCGGCGGAGGCGTTGACCGGGAGTCACCTTGATGAGGTGAAGA
AGATGGTGAAGGAGTTCAGGAAGCCGGTGGTTAAGCTTGGAGGAGAGACACTGACGG
TGTCTCAAGTGGCGGGGATCGCAGCTGCTAATGACAGCACCACCGTGACGGTGGAGCT
GTCGGAGGCGGCGAGGGCTGGAGTGAAGGCGAGCAGTGATTGGGTTATGGAGAGCAT
GAACAAAGGGACTGATAGTTATGGTGTTACCACCGGCTTCGGCGCCACCTCTCACCGG
AGAACCAAGCAAGGCGGTGCTTTACAGAAGGAGCTCATTAGATTTTTGAACGCCGGA
ATATTCGGTAATGGAACGGAATCTAGCCACACCCTACCACACTCAGCCACAAGAGCCG
CCATGCTTGTCAGAATCAACACCCTTCTCCAGGGATACTCCGGCATTCGATTCGAGAT
CTTGGAAGCTATCACCAAGTTCCTCAATAACAACATCACTCCTTGTTTACCCCTCCGTG
GGACCATCACCGCCTCCGGTGACCTTGTTCCATTATCTTACATCGCTGGTCTCCTCACC
GGCCGCCCAAACTCAAAGGCCGTCGGTCCAACCGGAGAAATCCTCAATGCCGAAAAG
GCATTCGCTGCTGCCGGTGTCGAAGGTGGTTTCTTCGAGTTACAGCCTAAAGAAGGGC
TAGCACTTGTTAACGGCACCGCCGTGGGATCCGGGATGGCGTCGATGGTTCTCTTCGA
AGCTAATGTACTTGCGTTGTTGTCGGAAGTGTTATCGGCGATCTTCGCTGAAGTTATGC
AAGGGAAGCCGGAGTTCACCGACCACTTAACACACAAATTGAAGCACCACCCCGGCC
AAATCGAAGCCGCCGCCATCATGGAATACATTTTGGACGGAAGCGATTACGTCAAGG
CGGCGCAAAAGGTACACGAAATGGACCCCTTACAGAAACCAAAACAAGACCGTTACG
CTCTCCGGACATCGCCCCAATGGCTCGGACCACACATTGAAGTAATCCGATCATCAAC
CAAAATGATCGAGAGGGAAATCAACTCCGTCAACGACAATCCACTCATCGACGTTTCC
 
AGAAACAAAGCCTTACACGGTGGCAACTTCCAAGGAACTCCAATCGGTGTTTCCATGG
ACAACACCCGTCTAGCCATCGCTTCCATCGGAAAACTCATGTTCGCCCAATTTTCTGAA
CTCGTTAACGATTTCTACAACAATGGATTACCGTCAAATCTCTCCGGCGGTCGTAACCC
TAGCTTGGATTACGGGTTCAAAGGTGGAGAAATCGCCATGGCTTCTTATTGCTCGGAG
CTCCAATTTCTCGCGAACCCAGTCACCAACCACGTTCAAAGCGCCGAACAACACAATC
AAGACGTTAACTCTTTGGGCTTAATTTCAGCGAGAAAAACTCAAGAAGCTGTCGAGAT
CTTAAAACTCATGTCCTCCACATATTTGGTCGCTCTATGCCAATCCATCGATTTACGCC
ATTTGGAAGAAAACATGAAATCGACAGTGAAGAACACCGTCAGCCAAGTCGCGAAAA
AGGTCCTAACAATGGGCGTCAACGGCGAGCTCCACCCTTCCAGATTCTGCGAAAAGGA
TTTACTCCGTGTTGTTGATCGCGAGTACGTCTTCGCCTACATCGACGACGCTTGCAGCG
CCACCTACCCGTTAATGCAAAAGCTCCGTCAAGTTCTCGTCGACCACGCCCTAAACAA
CGGCGAAACAGAGAAGAACACCAGCACCTCCATTTTCCAAAAGATCGCCACCTTCGA
AGACGAATTAAAAGCACTTTTACCGAAAGAAGTAGAAGCTGTCAGAATCGCGTACGA
AAACGACACATTACCGATCGCGAACAGAATCAAAGCCTGCAGATCGTACCCGTTGTAC
AGGTTTGTCAGGGAGGAGCTCGGCGGAGTGTTTTTGACCGGCGAGAAGGTGACGTCG
CCAGGGGAGGAGTTCGACAAGGTGTTTACCGCCATGTGCAAGGGTCAAATCATCGATC
CGTTGTTGGAATGTGTTGAAGGCTGGAATGGTGAACCTCTTCCGATTTGTTAG
**SEQ ID No:4**
   *> Cichorium endivia* L. modified PAL1 protein
   MENGNHVNGVVKEFCIKDPLN

## Claims

1. A mutant endive plant *(Cichorium endivia* L.) comprising a modified *PAL1* gene, wherein the modification leads to reduced browning of the endive leaves after the leaves having been in contact with hot water as compared to the leaves of a plant comprising the wild type *PAL1* gene, the wild type sequence of which has a sequence similarity of 95%, 96%, 97, 98%, 99% or 100% with **SEQ ID No:1,** wherein the modification of the *PAL1* gene is a premature stop codon in the coding sequence of said gene, and wherein the plant is not exclusively obtained by means of an essentially biological process.

2. The endive plant of claim 1, wherein the contact of the leaves with hot water is effected by submersion in hot water and wherein hot water is preferably water with a temperature of 85°C to 95°C, in particular about 90°C.

3. The endive plant as claimed in any one of the claims 1 to 2, wherein the premature stop codon is caused by the presence of a SNP form G to A at position 66 of **SEQ ID No:1.**

4. Seed comprising the modified *PAL1* gene as defined in any one of the claims 1 to 3, wherein the plant grown from the seed shows reduced browning of the endive leaves after the leaves having been in contact with hot water, in particular through submersion, wherein the seed is not exclusively obtained by means of an essentially biological process.

5. Propagation material capable of developing into and/or being derived from an endive plant as claimed in any one of the claims 1 to 3 or seed as claimed in claim 4, wherein the propagation material comprises the modified *PAL1* gene leading to reduced browning of the endive leaves after the leaves having been in contact with hot water, in particular through submersion, as claimed in any of the claims 1 to 4 and wherein the propagation material is selected from the group consisting of microspores, pollen, ovaries, ovules, embryos, embryo sacs, egg cells, cuttings, roots, root tips, hypocotyls, cotyledons, stems, leaves, flowers, anthers, seeds, meristematic cells, protoplasts, callus, and cells, or a tissue culture thereof, wherein the propagation material is not exclusively obtained by means of an essentially biological process.

6. A leaf of an endive plant as claimed in any of the claims 1 to 3, wherein the endive plant is not exclusively obtained by means of an essentially biological process.

7. Molecular marker comprising a SNP at position 66 of **SEQ ID No:1,** in particular a SNP at position 66, wherein the SNP is G to A.

8. Use of the molecular marker as claimed in claim 7 to identify a modification in the *PAL1* gene that leads to reduced browning of endive leaves after the leaves having been in contact with hot water, in particular through submersion.

9. A modified *PAL1* gene, the wild type sequence of which has a sequence similarity of 95%, 96%, 97, 98%, 99% or 100% with SEQ ID No:1, comprising a modification that leads to reduced browning of endive leaves after the leaves having been in contact with hot water, in particular through submersion, wherein the modification of the *PAL1* gene is a premature stop codon in the coding sequence of said gene.

10. A modified *PAL1* gene as claimed in claim 9, wherein the modification results in a premature stop codon.

11. A modified *PAL1* gene as claimed in claim 10, wherein the nucleotide of said gene at position 66 of **SEQ ID No:1** is modified from G to A.

12. Method for obtaining an endive plant with reduced browning of the leaves after the leaves having been in contact with hot water, in particular through submersion, comprising reducing the endogenous level of the PAL1 protein in the plant, wherein the wild type sequence of the PAL1 protein has a sequence similarity of 95%, 96%, 97, 98%, 99% or 100% with the protein sequence represented by **SEQ ID No: 2.**

13. Method according to claim 12, wherein reducing the endogenous level of the PAL1 protein is effected by mutagenic treatment of the PAL1 gene, by CRISPR, a chemical agent, or radiation, gene silencing, RNAi, or a combination thereof.

## Patentansprüche

1. Mutanten-Endivienpflanze *(Cichorium endivia L.),* umfassend ein modifiziertes PAL1-Gen, wobei die Modifikation zu einer reduzierten Bräunung der Endivienblätter führt, nachdem die Blätter in Kontakt mit Heißwasser waren, verglichen mit den Blättern einer Pflanze, umfassend das Wildtyp-*PAL1*-Gen, dessen Wildtyp-Sequenz eine Sequenzähnlichkeit von 95 %, 96 %, 97, 98 %, 99 % oder 100 % mit **SEQ ID No:1** aufweist, wobei die Modifikation des *PAL1-*Gens ein vorzeitiges Stopcodon in der codierenden Sequenz des Gens ist, und wobei die Pflanze nicht ausschließlich mittels eines im Wesentlichen biologischen Prozesses erhalten wird.

2. Endivienpflanze nach Anspruch 1, wobei der Kontakt der Blätter mit Heißwasser durch ein Eintauchen in Heißwasser bewirkt wird und wobei Heißwasser vorzugsweise Wasser mit einer Temperatur von 85 °C bis 95 °C ist, insbesondere etwa 90 °C.

3. Endivienpflanze nach einem der Ansprüche 1 bis 2, wobei das vorzeitige Stopcodon durch die Gegenwart eines SNP von G bis A bei Position 66 von **SEQ ID No:1** verursacht wird.

4. Samen, umfassend das modifizierte *PAL1-*Gen wie in einem der Ansprüche 1 bis 3 definiert, wobei die Pflanze, die aus dem Samen gezogen wird, eine reduzierte Bräunung der Endivienblätter zeigt, nachdem die Blätter in Kontakt mit Heißwasser waren, insbesondere durch Eintauchen, wobei der Samen nicht ausschließlich mittels eines im Wesentlichen biologischen Prozesses erhalten wird.

5. Vermehrungsmaterial, das in der Lage ist, sich zu einer Endivienpflanze zu entwickeln und/oder von einer Endivienpflanze abgeleitet zu sein, nach einem der Ansprüche 1 bis 3, oder Samen nach Anspruch 4, wobei das Vermehrungsmaterial das modifizierte PAL1-Gen umfasst, das zu einer reduzierten Bräunung der Endivienblätter führt, nachdem die Blätter mit Heißwasser in Kontakt waren, insbesondere durch Eintauchen nach einem der Ansprüche 1 bis 4, und wobei das Vermehrungsmaterial ausgewählt ist aus der Gruppe, bestehend aus Mikrosporen, Pollen, Fruchtknoten, Samenanlagen, Keimlingen, Embryosäcken, Eizellen, Stecklingen, Wurzeln, Wurzelspitzen, Hypokotylen, Keimblättern, Stängeln, Blättern, Blüten, Staubbeuteln, Samen, meristematischen Zellen, Protoplasten, Callus und Zellen, oder einer Gewebekultur davon, wobei das Vermehrungsmaterial nicht ausschließlich mittels eines im Wesentlichen biologischen Prozesses erhalten wird.

6. Blatt einer Endivienpflanze nach einem der Ansprüche 1 bis 3, wobei die Endivienpflanze nicht ausschließlich mittels eines im Wesentlichen biologischen Prozesses erhalten wird.

7. Molekularer Marker, umfassend einen SNP an Position 66 von **SEQ ID No:1,** insbesondere einen SNP an Position 66, wobei der SNP G bis A ist.

8. Verwendung des molekularen Markers nach Anspruch 7, um eine Modifikation in dem *PAL1-*Gen zu identifizieren, die zu einer reduzierten Bräunung von Endivienblättern führt, nachdem die Blätter mit Heißwasser in Kontakt waren, insbesondere durch Eintauchen.

9. Modifiziertes *PAL1-Gen,* dessen Wildtyp-Sequenz eine Sequenzähnlichkeit von 95 %, 96 %, 97, 98 %, 99 % oder 100 % mit SEQ ID No:1 aufweist, umfassend eine Modifikation, die zu einer reduzierten Bräunung von Endivienblättern führt, nachdem die Blätter mit Heißwasser in Kontakt waren, insbesondere durch Eintauchen, wobei die Modifikation des PAL1-Gens ein vorzeitiges Stopcodon in der codierenden Sequenz des Gens ist.

10. Modifiziertes PAL1-Gen nach Anspruch 9, wobei die Modifikation zu einem vorzeitigen Stopcodon führt.

11. Modifiziertes *PAL1*-Gen nach Anspruch 10, wobei das Nukleotid des Gens an Position 66 von **SEQ ID No:1** von G bis A modifiziert ist.

12. Verfahren zum Erhalten einer Endivienpflanze mit reduzierter Bräunung der Blätter, nachdem die Blätter mit Heißwasser in Kontakt waren, insbesondere durch Eintauchen, umfassend das Reduzieren des endogenen Niveaus des PAL1-Proteins in der Pflanze, wobei die Wildtyp-Sequenz des PAL1-Proteins eine Sequenzähnlichkeit von 95 %, 96 %, 97, 98 %, 99 % oder 100 % mit der Proteinsequenz aufweist, die durch **SEQ ID No:2** dargestellt ist.

13. Verfahren nach Anspruch 12, wobei das Reduzieren des endogenen Niveaus des PAL1-Proteins durch mutagene Behandlung des PAL1-Gens, durch CRISPR, ein chemisches Mittel oder Bestrahlung, Genstilllegung, RNAi oder eine Kombination davon bewirkt wird.

## Revendications

1. Plant d'endive mutante *(Cichorium endivia L.)* comprenant un gène *PAL1* modifié, dans lequel la modification entraîne une réduction du brunissement des feuilles d'endive après que les feuilles ont été en contact avec de l'eau chaude par comparaison avec les feuilles d'un plant comprenant le gène *PAL1* de type sauvage, dont la séquence de type sauvage présente une similarité de séquence de 95 %, 96 %, 97 %, 98 %, 99 % ou 100 % avec **SEQ ID No : 1,** dans lequel la modification du gène *PAL1* est un codon d'arrêt prématuré dans la séquence codante dudit gène, et dans lequel le plant n'est pas exclusivement obtenu au moyen d'un procédé essentiellement biologique.

2. Plant d'endive selon la revendication 1, dans lequel le contact des feuilles avec de l'eau chaude est effectué par immersion dans de l'eau chaude, et dans lequel l'eau chaude est de préférence de l'eau à une température de 85 °C à 95 °C, en particulier d'environ 90 °C.

3. Plant d'endive selon l'une quelconque des revendications 1 à 2, dans lequel le codon d'arrêt prématuré est provoqué par la présence d'une forme SNP G à A à la position 66 de **SEQ ID No : 1.**

4. Semence comprenant le gène *PAL1* modifié tel que défini dans l'une quelconque des revendications 1 à 3, dans laquelle le plant cultivé à partir de la semence présente un brunissement réduit des feuilles d'endive après que les feuilles ont été en contact avec de l'eau chaude, en particulier par immersion, dans laquelle la semence n'est pas exclusivement obtenue au moyen d'un procédé essentiellement biologique.

5. Matériau de multiplication capable de se développer en un plant d'endive et/ou d'être dérivé d'un plant d'endive selon l'une quelconque des revendications 1 à 3 ou semence selon la revendication 4, dans lequel le matériau de multiplication comprend le gène *PAL1* modifié entraînant une réduction du brunissement des feuilles d'endive après que les feuilles ont été en contact avec de l'eau chaude, en particulier par immersion, tel que revendiqué dans l'une quelconque des revendications 1 à 4, et dans lequel le matériau de multiplication est choisi dans le groupe constitué de microspores, pollen, ovaires, ovules, embryons, sacs embryonnaires, cellules d'œuf, boutures, racines, extrémités de racines, hypocotyles, cotylédons, tiges, feuilles, fleurs, anthères, semences, cellules méristématiques, protoplastes, cals et cellules, ou culture tissulaire de ceux-ci, dans lequel le matériau de multiplication n'est pas obtenu exclusivement au moyen d'un procédé essentiellement biologique.

6. Feuille d'un plant d'endive selon l'une quelconque des revendications 1 à 3, dans laquelle le plant d'endive n'est pas exclusivement obtenu au moyen d'un procédé essentiellement biologique.

7. Marqueur moléculaire comprenant un SNP à la position 66 de **SEQ ID No : 1,** en particulier un SNP à la position 66, dans lequel le SNP est de G à A.

8. Utilisation du marqueur moléculaire selon la revendication 7 pour identifier une modification dans le gène *PAL1,* laquelle entraîne une réduction du brunissement des feuilles d'endive après que les feuilles ont été en contact avec de l'eau chaude, en particulier par immersion.

9. Gène *PAL1* modifié, dont la séquence de type sauvage présente une similarité de séquence de 95 %, 96 %, 97 %, 98 %, 99 % ou 100 % avec SEQ ID No : 1, comprenant une modification qui entraîne une réduction du brunissement des feuilles d'endive après que les feuilles ont été en contact avec de l'eau chaude, en particulier par immersion, dans lequel la modification du gène *PAL1* est un codon d'arrêt prématuré dans la séquence codante dudit gène.

10. Gène *PAL1* modifié selon la revendication 9 dans lequel la modification aboutit à un codon d'arrêt prématuré.

11. Gène *PAL1* modifié selon la revendication 10, dans lequel le nucléotide dudit gène à la position 66 de **SEQ ID No : 1** est modifié de G à A.

12. Procédé d'obtention d'un plant d'endive avec un brunissement réduit des feuilles après que les feuilles ont été en contact avec de l'eau chaude, en particulier par immersion, comprenant la réduction du niveau endogène de la protéine PAL1 dans le plant, dans lequel la séquence de type sauvage de la protéine PAL1 a une similarité de séquence de 95 %, 96 %, 97 %, 98 %, 99 % ou 100 % avec la séquence protéique représentée par **SEQ ID No : 2.**

13. Procédé selon la revendication 12, dans lequel la réduction du niveau endogène de la protéine PAL1 est effectuée par un traitement mutagène du gène PAL1, par CRISPR, un agent chimique, ou par rayonnement, silençage de l'expression génique, ARNi ou une combinaison de ceux-ci.
